# EUROPEAN PATENT SPECIFICATION

(11) **EP 4 225 176 B1**
(45) Date of publication and mention of the grant of the patent: **29.01.2025**
(21) Application number: 21794246.5
(22) Date of filing: 08.10.2021
(51) Int. Cl.: A61B 17/70, A61B 90/00

(54) **CONNECTOR FOR SPINAL CORRECTION**
VERBINDER ZUR WIRBELSÄULENKORREKTUR
CONNECTEUR POUR CORRECTION VERTÉBRALE

(30) Priority: 08.10.2020 NL 2026643
(43) Date of publication of application: 16.08.2023
(73) Proprietor: UMC Utrecht Holding B.V., 3584 CS Utrecht (NL)
(72) Inventor: KRUYT, Moyo C., 3584 CM Utrecht (NL); CASTELEIN, René M., 3584 CM Utrecht (NL)
(74) Representative: Arnold & Siedsma
(86) International application number: PCT/NL2021/050611
(87) International publication number: WO 2022/075849

(56) References cited:
- EP-A1- 3 406 212
- WO-A1-2010/030906
- US-A1- 2009 204 156
- US-A1- 2010 063 545

## Description

The present invention relates to a connector for connecting an anchoring device to a rod in a spinal correction device, a spinal correction system comprising the said connector and a kit of parts.

### BACKGROUND OF THE INVENTION

Spine surgeons can use many types of systems for spinal fixation. A known growth-guiding system for instance comprises a rod coupled to one end of the spine, a connector slidably coupled to said rod which connects to an anchoring device on the other end of the spine. The connector then allows relative movement of the rod and thus between the connection point at the lower end of the spine and the anchoring point. It is also known to use helical springs positioned around the rod to allows continuous distraction forces, which stimulates spinal growth.

A connector of the type claimed is known from the US 2010/063545 A1, from which the preamble of claim 1 derives.

### SUMMARY OF THE INVENTION

The present invention is defined in claim 1 while preferred embodiments are set forth in the dependent claims.

Associated surgical methods are also described herein to aid understanding the invention. These methods do not form part of the claimed invention.

The object of the invention is to provide an improved connector for slidably connecting an anchoring device to a rod in a spinal correction system.

This object is met by a connector for slidably connecting an anchoring device to a rod in a spinal correction system, comprising a body, wherein the body has a first tubular opening, a coupling mechanism for coupling the body to the anchoring device, a guiding mechanism provided in said first tubular opening and arranged to guide the rod in a sliding movement along a longitudinal direction of the rod, wherein said guiding mechanism has at least two guiding portions provided at a mutual distance in said first tubular opening and is further arranged to prevent any movement, with respect to the rod, other than a translation along the longitudinal direction of the rod and a rotation around said longitudinal direction of the rod.

From the daily practice of orthopaedic surgeons came the observation that existing growing spine solutions suffer from the issue of proximal junctional kyphosis (PJK), where a moment in a sagittal plane is applied to the spinal correction system. By providing a connector for slidably coupling to a rod, while substantially preventing any relative rotation in the sagittal plane, the problem of kyphosis is believed to be significantly reduced.

Preferably, the guiding mechanism is arranged to prevent any movement with respect to the rod other than a movement with respect to said longitudinal direction of the rod and preferably axial rotation around said longitudinal direction. Generally, it is preferred if the guiding mechanism is arranged to prevent any rotation of the body with respect to the rod in any plane containing the longitudinal direction or axis of the rod. Rotation in for instance the sagittal plane and the coronal plane is thus prevented.

The coupling mechanism is arranged to couple to another orthopaedic device, for instance the anchoring device as mentioned. The coupling mechanism may also be formed integrally with another constructional element, such as an anchoring device. Rotation of such a device in the sagittal and coronal planes is then prevented. As the coupling mechanism, or another device as mentioned above, is preferably rigidly coupled to the body, also the relative movement of the coupling mechanism, and therewith any coupled other device, is fixed. Also such a device is thus not allowed to rotate in the sagittal or coronal plane, or any other plane containing the longitudinal direction or axis as mentioned above.

As mentioned, the guiding mechanism is arranged to prevent any movement, preferably with respect to the rod, other than a translation along or rotation around the longitudinal direction of the rod. In the alternative, only translation is allowed. The connector is then only allowed to translate longitudinally along the length of the rod. Any rotation of the body in other planes is then prevented with respect to the rod.

It is however preferred if guiding mechanism is further, or alternatively only, arranged to also allow a rotation around said longitudinal direction of the rod. In this way, rotational and axial mobility may be maintained while constraining any other movement, including a movement in a sagittal plane associated with kyphosis. As mentioned, rotation of the body with respect to the rod in other planes, such as the coronal and sagittal planes, is preferably also prevented.

It is remarked that when a curved rod is used, as will be explained in greater detail below, longitudinal direction of the rod may vary along the length, also within the connector. The guiding mechanism is then arranged to prevent any movement other than a movement with respect to an average longitudinal direction, as seen along the length, or to substantially prevent any movement as described. The guiding mechanism is arranged to allow relative movement with respect to the rod in only one degree of freedom, preferably sliding of the connector on the rod, i.e. in the longitudinal or axial direction. The guiding mechanism is further arranged to further allow movement in a second degree of freedom, i.e. rotation or roll around the rod. Thus, the guiding mechanism is arranged to only allow movement of the body of the connector, and therewith the coupling mechanism, in only two degrees of freedom, i.e. axial sliding along and rotation around the rod in the transverse plane.

According to a preferred embodiment, the body has a first tubular opening and the guiding mechanism is provided in the first tubular opening. The rod may thus be constrained by the geometric properties of the connector.

According to a preferred embodiment, the guiding mechanism has at least two guiding portions provided at a mutual distance in said first tubular opening. The rod may be constrained by aligned guiding portions acting as a constraining conduit for the rod.

Preferably, the at least two guiding portions are provided at the end parts of the first tubular opening. This allows easy insertion of the guiding portions in the body and the physical size of the connector may be kept to a minimum. This further allows the receiving and guiding rods having more pronounced curvatures, as will be further explained below.

According to a preferred embodiment, the first tubular opening has a first internal diameter, the guiding portions have a second internal diameter, and the first internal diameter is larger than the second internal diameter. The first tubular opening may accommodate a rod with a curvature, in particular a rod contoured into the desired shape in both the coronal and sagittal plane.

According to a preferred embodiment, each guiding portion has a bearing or set of bearings and a bearing holder. The bearing portion is preferably a bearing mounted on the bearing holder and allowed to rotate with respect to said holder. Preferably, the bearing is rotatable around a plurality of axis with respect to said holder. Sliding of the rod is allowed at low friction while preventing metal debris and metallosis or metal poisoning, due to its specific lining. Curved rods are further efficiently received and guided.

According to a preferred embodiment, the guiding portions are spherical bearings. A rod with a curvature may then easily be accommodated through the ends of the tubular opening, since spherical bearings allow three rotations around roll, yaw and pitch axes.

For efficient fabrication, it is preferred if the guiding portions are slid inside the body through slot openings in the body.

According to a preferred embodiment, the guiding portions are slid inside the body via the ends of the first tubular opening. Bearing holders may thus be inserted in the body while the body may retain its integrity and while space for a locking mechanism in the middle part of the connector may be preserved.

According to a preferred embodiment, the guiding portions are fixed using one of a pin mechanism, a thread mechanism, a bayonet mechanism, a snap fit mechanism. More in particular, the bearing holders are fixed using one of a pin mechanism, a thread mechanism, a bayonet mechanism, a snap fit mechanism. In this way, bearings and their bearing holders may be inserted in the body in a tool less manner.

According to a preferred embodiment, a spring is preferably provided and preferably there is further a docking provided for said spring, wherein said spring is arranged for providing a distraction force along the longitudinal direction of the rod. In this way, distraction may be implemented in combination with sagittal control. A first end of the spring may abut the body of the connector, for instance at said docking, while a second end of the spring may abut a stop provided on the rod. Preferably, the spring is arranged around the rod.

According to a preferred embodiment, the body has a locking mechanism for locking the rod in a fixed position with respect to the body. In this way, a permanent fixation may be achieved, which is particularity useful when used in combination with a spring while implanting such a system.

According to a preferred embodiment, the coupling mechanism has a second tubular opening for receiving a stationary rod. Such a rod can be used for fixation to at least one, preferably a plurality of vertebrae, for instance using bone pins. Preferably, said second tubular opening has a direction substantially parallel to the direction of the first tubular opening. In this way, the transmission of a torque between the rods may be minimized.

According to a preferred embodiment, the coupling mechanism has a locking mechanism for locking the stationary rod with respect to the coupling mechanism. In this way, a kyphosis moment on the stationary rod may be transmitted to the connector.

The object of the invention is also met by a connector assembly for spinal correction comprising a connector as described above and an anchoring device for anchoring to the spine, said anchoring device being coupled to the connector via the coupling mechanism of the connector. In this way, kyphosis of a part of the spine may be controlled.

According to a preferred embodiment, the anchoring device has a stationary rod. In particular, the anchoring device has one or more anchoring elements for anchoring in one or more vertebrae. More in particular, the stationary rod is fixed by two anchoring elements in two neighbouring vertebrae. In this way, a kyphosis force on a part, mostly a superior part, of the spine may be controlled.

The object of the invention is also met by a spinal correction system comprising a rod for spinal correction and at least one connector according to any of the above preferred embodiments. In this way, a complete system for growth and/or distraction systems controlling kyphosis is provided.

According to a preferred embodiment, the system further comprises an anchoring device to form with the at least one connector a connector assembly according to any of the above embodiments. In particular, one or more anchors are further provided for anchoring the rod in the spine. In this way, kyphosis of a part, mostly a superior part, of the spine may be controlled.

According to the invention, is further provided a kit of parts comprising at least two of a connector, an anchoring device and a rod.

Also described herein, but not claimed, is a method for providing a spinal correction to a patient, comprising the steps of providing a rod for attachment to the spine of the patient, providing an anchoring device for attachment to the spine of the patient, providing a connector according to any of the above preferred embodiments for connection to the rod and the anchoring device, and coupling the connector to the rod. According to a preferred embodiment, the method further comprises coupling the connector to the anchoring device.

The method preferably comprises a step of attaching the rod to the spine, for instance using bone screws or bone pins. Providing the anchoring device may comprise attaching the anchoring device to the spine, in particular to at least one vertebra. The anchoring device may be unitary with the connector, in the alternative the connector can be coupled to the anchoring device.

The connector is preferably coupled to the rod by sliding the connector, preferably using the guiding mechanism as described above, over the rod. After connection to the anchoring device, the anchoring device can be attached to the spine. Or the connector can be attached to the anchoring device once the anchoring device is attached to the spine.

In particular the connector or system as described may be used for the following non exhaustive list of spinal applications:
- as a "soft landing" in long trajectory segmental fusions, for example in degenerative scoliosis. In that application an additional stopper ring can be used at the end of the rod without interposition of a small absorption tube or spring;
- to guide spinal stiffening in degenerative disc disease to prevent or treat degenerative scoliosis;
- to allow load sharing of the axial forces between instrumentation and the vertebrae in long trajectory stabilizations, this will prevent disuse osteoporosis;
- to maintain mobility between instrumented segments which do not have to fuse, but need to support sagittal stability, for example, but not limited to, in fracture healing;
- to allow passive growth in growth guiding systems. Due to the option to combine several vertebrae and or lamina and ribs, the anchors can be adapted to the specific needs and are much stronger;
- to allow active growth /distraction in hybrid growing rod constructs such as in combination with Magnetically Controlled Growing Rods (MCGR);
- in combination with a stop ring at the apical side of the connector. In such a way the device allows to maintain rotational mobility in active growth or supporting systems like traditional growing rods and MCGR;
- the stop ring at the end of the rod can prevent the rod coming loose from the connector.

Thus, a method is provided according to any of the above applications, including the step of providing a connector or system as mentioned above.

Although arranged for spinal correction, the connector could be arranged for other orthopaedic applications related to bones or joints in general. For instance, the connector could be used for slidably connecting to rods in system connected to hands and feet. The principle of the connector described here is therefore not limited to spinal correction insofar as the concept of an orthopaedic connector constraining a moment in a given plane as disclosed here may also be declined accordingly for other types of orthopaedic applications.

It is noted that various known planes and directions will be referred to in the present application and are here explained for the ease of the reading. A sagittal plane refers to a plane defined by two axes, one drawn between a head (superior) and tail (inferior) of the body, and one drawn between a back (posterior) and front (anterior) of the body. A coronal plane refers to a plane defined by two axes, one drawn between a center (medial) to side (lateral) of the body, and one drawn between a head (superior) and tail (inferior) of the body. A transverse plane refers to a plane defined by two axes, one drawn between a back and a front of the body, and one drawn between a center and a side of the body.

The present invention will now be described in more detail, with reference to the appended drawings showing currently preferred embodiments of the invention, wherein:
- Figure 1 illustrate a view of a complete spinal correction system comprising the connector;
- Figure 2 illustrates a cross-section of the connector according to an embodiment with a straight rod;
- Figure 3 illustrates a cross-section of the connector according to an embodiment with a rod having a curvature;
- Figure 4 illustrates a perspective view and three cross-sections of the connector according to an alternative embodiment with a connector body having spherical bearing holders;
- Figure 5 illustrates a cross-section of the connector according to an alternative embodiment with a connector body configured having a pin mechanism for locking bearing holders;
- Figure 6 illustrates a cross-section of the connector according to an alternative embodiment with a connector body configured having a thread mechanism for locking bearing holders;
- Figure 7 illustrates a cross-section of the connector according to an alternative embodiment with a connector body configured having a bayonet mechanism for locking bearing holders;
- Figure 8 illustrates a cross-section of the connector according to an alternative embodiment with a connector body provided with a locking ring mechanism for locking bearing holders;
- Figure 9 illustrates a cross-section of the connector according to an alternative embodiment with a connector body having a snap fit mechanism for locking bearing holders;
- Figure 10 illustrates a cross-section of the connector according to an alternative embodiment with a connector body configured for interlocking bearing holders.

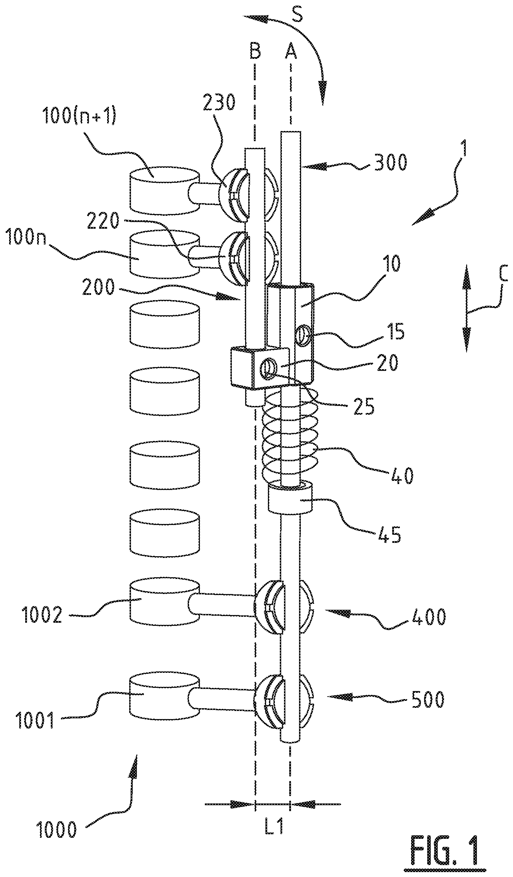

Figure 1 shows a spinal correction system 1 comprising a connector 100, an anchoring device 200, a rod 300 and anchoring elements 400 and 500 for anchoring to the spine 1000. Each element of the system will now be described successively.

As shown in Figure 1, the rod 300 is an elongate member, having a cylindrical shape and being secured to the spinal column 1000 via two anchoring elements 400, 500. The anchoring elements 400, 500 are mounted, or fixed to two vertebrae 1001, 1002 located at a first position along the spine 1000, in an inferior part of the spinal column 1000. Optionally the rod 300 may be anchored at additional anchoring points. The anchoring elements 400, 500 are further adapted to receive and secure the rod 300, such that the rod 300 is made solidary with the two vertebrae 1001 and 1002.

The rod 300 in Figure 1 is further illustrated as having a round cross section with a diameter D1. Yet other embodiments may be envisaged with a rod having a different contour and in particular with a rod having one or more flat portions. As illustrated in Figure 1, the rod 300 is a straight rod having an axis of symmetry. In such a case, a longitudinal direction A of the rod 300 coincides with the longitudinal axis of the rod 300.

The anchoring device 200 comprises a stationary rod 210 extending along a longitudinal direction B and two anchoring elements 220 and 230. The anchoring elements 220 and 230 may be similar to the anchoring elements 400 and 500 and may be mounted or fixed to two vertebrae 100n and 100(n+1) located at a second position along the spine 1000, in a superior part of the spinal column 1000. The second position is in this example located above the first position along the spinal column 1000. Optionally the stationary rod 210 may be anchored at additional anchoring points.

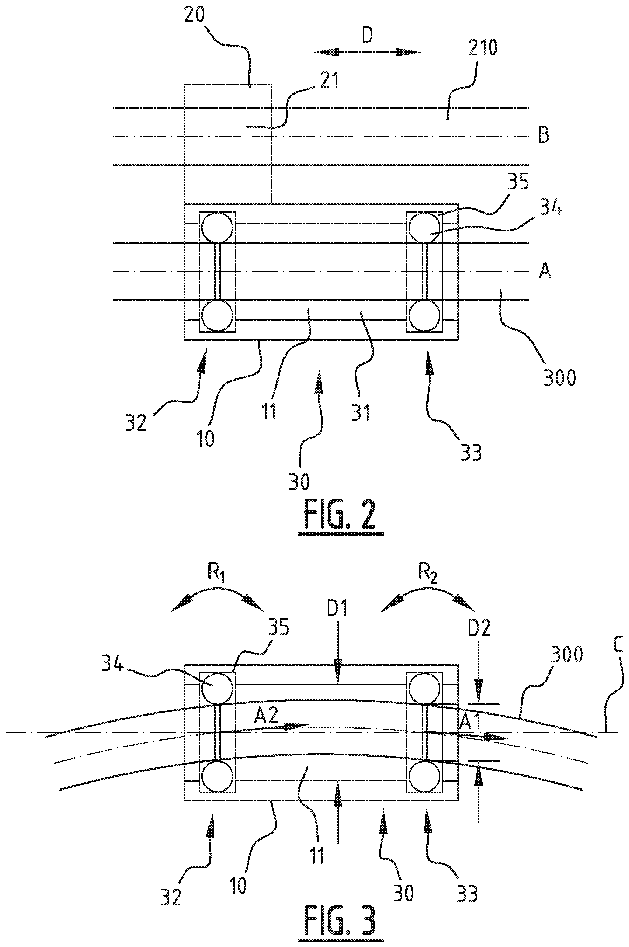

The connector 100 comprises a body 10, a coupling mechanism 20 for coupling the body 10 to the anchoring device 200, and a guiding mechanism 30. The guiding mechanism 30 (see also figures 2a and 2b) is configured to guide the rod 300 in a sliding movement along the longitudinal direction A of the rod 300, and is further arranged to prevent any movement with respect to the rod other than a movement with respect to said longitudinal direction A of the rod 300.

The body 10 illustrated in Figure 1 has an elongated tubular shape with a longitudinal direction (see arrow C in figure 1) matching the longitudinal direction A of the axis of the rod 300, such that the rod 300 may cross right through the body 10 along the longitudinal direction A. Although represented as rather a tubular shaped element, other shapes of a body may be envisaged for receiving the rod 300 in the body 10.

The connector 100 is such that when assembled in the system of Figure 1 any movement in a sagittal plane (see arrow S in figure 1 illustrating this movement) may be constrained. When the rod 300 is mounted in the connector 100, or in other words, when the connector 100 is coupled to the rod 300, the connector 100 may slide freely along the longitudinal direction A of the rod 300 while any other movement is prevented. In particular any movement in a sagittal plane is prevented. Only a translation along the direction A and a roll rotation movement around the axis of the rod are allowed by the guiding mechanism 30 of the connector 100. In this way, rotational and axial mobility for growth or distraction is maintained while preventing the issue of kyphosis, particularly present in cases of growing or distraction systems.

For the option where some distraction between the lower vertebrae 1001, 1002 and the upper vertebrae 100n, 100(n+1) is desired, a spring mechanism 40 may be provided around the rod 300 for constraining the translation movement of the connector 100 along the rod 300 by exerting a compression effort between a stopper element 45 and the body 10 of the connector 100. The stopper element can be used proximal and distal depending on the desired function.

In the embodiment of Figure 1, the coupling mechanism 20 is integrally attached to the body 10 to form an L-shaped connector 100. The long side of the L-shape corresponds substantially to the body 10 of the connector 100 and extends in the longitudinal direction A of the rod 300. The short side of the L-shaped connector 100 corresponds substantially to the coupling mechanism 20. Alternatively the coupling mechanism 20 may provide coupling to an additional element (not represented in any of the figures) configured solely for anchoring and/or to receive and secure the stationary rod 210 or any other orthopaedic device.

The coupling mechanism 20 is configured to receive and secure the stationary rod 210, such that the stationary rod 210 is made solidary with the coupling mechanism 20 and thus with the connector 100. The coupling mechanism 20 is configured to secure the stationary rod 210 in a direction B. The direction B may be substantially parallel to the direction A such that the rod 300 and the rod 210 are substantially parallel to each other and at a mutual lateral distance L1 chosen to minimize the transmission of a torque between the rods 210 and 300. The rod 300 is further positioned next to the anchoring device 200 to preserve mobile segments of the spine as much as possible

The coupling mechanism 20 may comprise a locking mechanism 25 for locking the stationary rod 210 with respect to the coupling mechanism 20. A screw may for instance be provided in an opening in the coupling mechanism 20 for securing the stationary rod 210 to the coupling mechanism 20.

Similarly the body 10 may comprise a locking mechanism 15 for locking the rod 300 with respect to the body 10. A screw may for instance be provided in an opening in the body 10 for securing the rod 300 to the body 10.

Figure 2a illustrates a cross-section of the connector 100 connected to a straight rod 300. In particular Figure 2a shows how the body 10 has a first tubular opening 11 extending through the body 10 in a longitudinal direction of the body 10 (coinciding with direction A in figure 2a), creating a receptacle for the rod 300. The guiding mechanism 30 is then provided in the first tubular opening 11 for guiding the rod 300 in a sliding movement along the longitudinal direction A of the rod 300.

As illustrated the guiding mechanism 30 provided inside the tubular opening 11 has at least two guiding portions 32, 33 provided at a mutual distance D in said first tubular opening 11. In particular the two guiding portions 32, 33 are provided at or near the end parts of the first tubular opening 11. Each guiding portion 32, 33 comprises an annular bearing 34 housed in a bearing holder 35. Each annular bearing 34 can rotate in its bearing holder such that the rod 300 may slide and roll with respect to the direction A. The bearings 34 may be spherical bearings.

The cross section of the coupling mechanism 20 shows further a second tubular opening 21 in the coupling mechanism 20 for receiving the stationary rod 210. The second tubular opening 21 has a longitudinal direction B substantially parallel to the longitudinal direction of the first tubular opening 11.

Figure 2b illustrates a cross-section of the connector when connected to a rod 300 having a curvature. Of significance is that the bearings 34 are spherical bearings allowing a rotation according to a roll, pitch and yaw rotation axis, see arrows R1 and R2 in figure 2b, allowing the bearings to adjust to the surface of the curved rod 300. The first internal diameter D1 of the tubular opening 11 is larger than the second internal diameter D2 of the bearings 34 to allow the movement of the rod having a curvature in the region there between. The second internal diameter D2 is dimensioned relative to the diameter of the rod 300 to allow a translation with a predetermined friction. In this case the sliding movement of the rod is guided locally at each guiding portion 32, respectively 33 in a longitudinal direction A1, respectively A2. It is noted that the longitudinal directions A1 and A2 of the sliding movement of the rod at the guiding portions 32 and 33 are substantially the same as the longitudinal direction C of the first tubular opening 11.

Figure 3 illustrates a perspective view and three cross-sections of a connector 100 with a connector body 10 comprising two bearing holders 35 formed as spherical cavities inside the tubular opening 11 in the body 10. At the extremities 10a and 10b of body 10 along the longitudinal direction A, the tubular opening 11 mouths into an oval shape having a long axis dimension D3 greater than a short side dimension D4, wherein D3 and D4 are chosen relative to the external diameter of the bearings 34, such that D4 is greater than the external diameter of the bearings 34 and D4 is smaller than the external diameter of the bearings 34. The oval shaped holes at the ends 10a and 10b of the body allow the insertion and the shape locking of annular bearings 34 in the spherical bearing holder cavities 35.

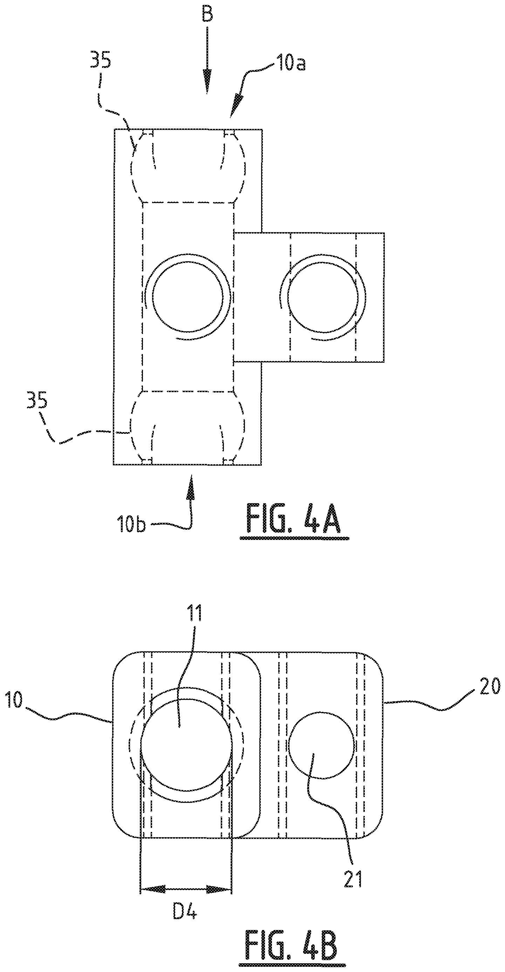

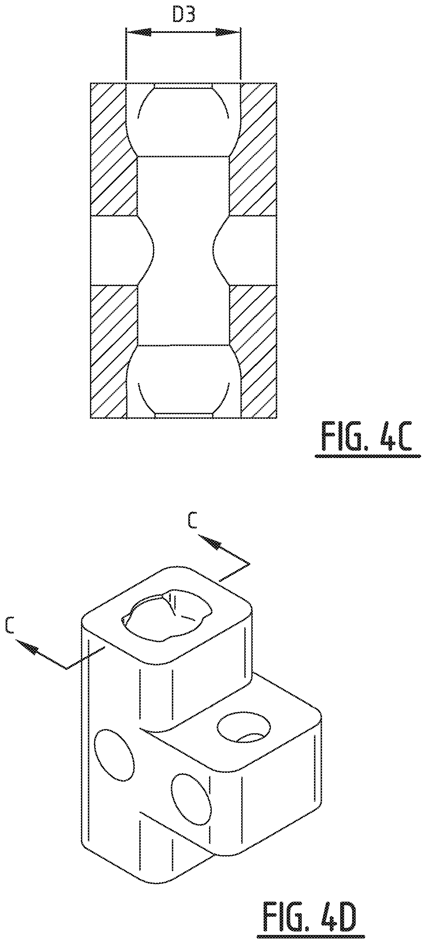

Figures 4-10 illustrate alternative embodiments where the bearing holders 35 are separate elements form the body 10. In particular the embodiments of Figures 4-10 show alternatives where the bearings 34 and their bearings holders 35 are slid inside the body 10 via the ends of the first tubular opening 11.

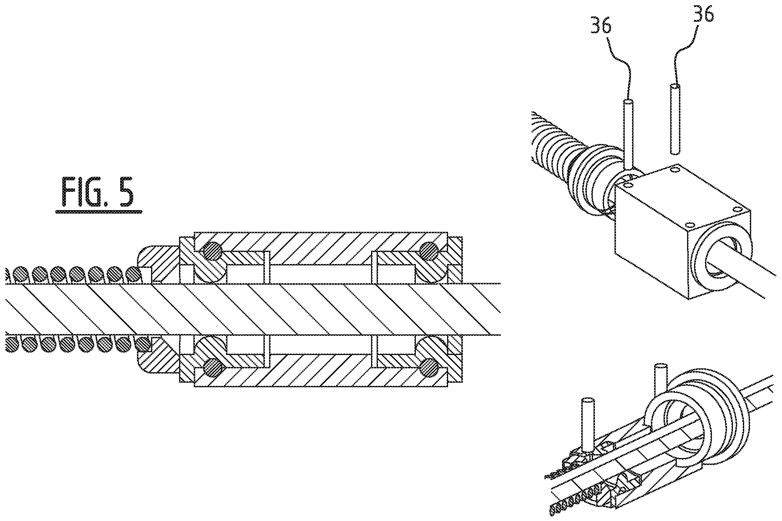

Figure 5 illustrates a cross-section of the connector according to an alternative embodiment of the invention with a connector body configured having a pin mechanism 36 for locking bearing holders 35. First the bearing holders 35 housing the bearings 34 are slid from each end of the body 10 inside the tubular opening 11, then pins 36 are engaged from the outside of the housing into holes provided in the body 10 and the bearing holders 35 to lock the bearing holders 35 inside the body 10.

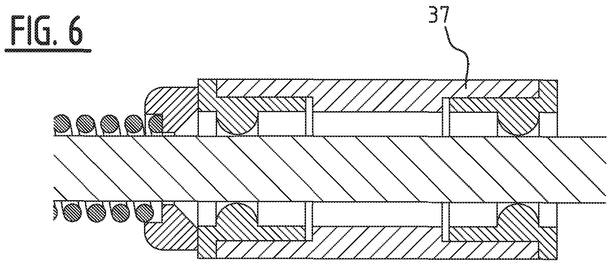

Figure 6 illustrates a cross-section of a connector with a connector body 10 configured having a thread mechanism 37 for locking bearing holders 35. Each bearing holder 35 is provided with a thread 37 mating a thread provided on the inner side of the body 10 such that the bearing holders 35 and the bearings 34 may be presented via the ends and fixed by rotation inside the body 10.

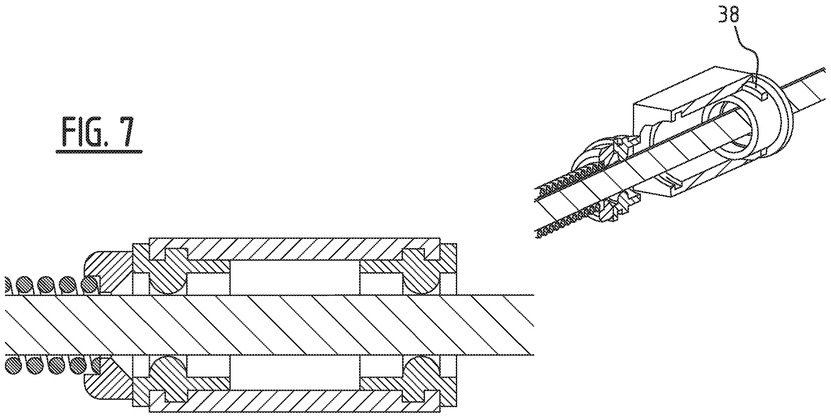

Figure 7 illustrates a cross-section of the connector with a connector body 10 configured having a bayonet mechanism 38 for locking bearing holders 35. Each bearing holder 35 is provided with a bayonet 38 mating a bayonet thread provided on the inner side of the body 10 such that the bearing holders 35 and the bearings 34 may be presented via the ends and fixed by rotation inside the body 10.

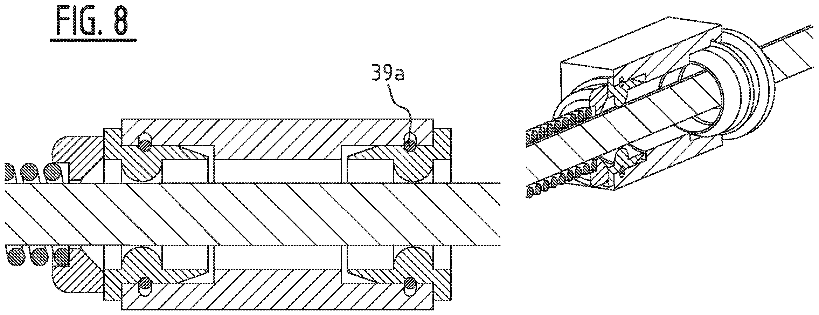

Figure 8 illustrates a cross-section of the connector with a connector body 10 provided with a locking ring mechanism 39a for locking bearing holders 35. Each bearing holder 35 is provided with a locking ring 39a meant to lock into a recess provided on the inner side of the body 10 such that the bearing holders 35 and the bearings 34 may be presented via the ends and snap-fitted when the locking ring 38a falls into the recess inside the body 10.

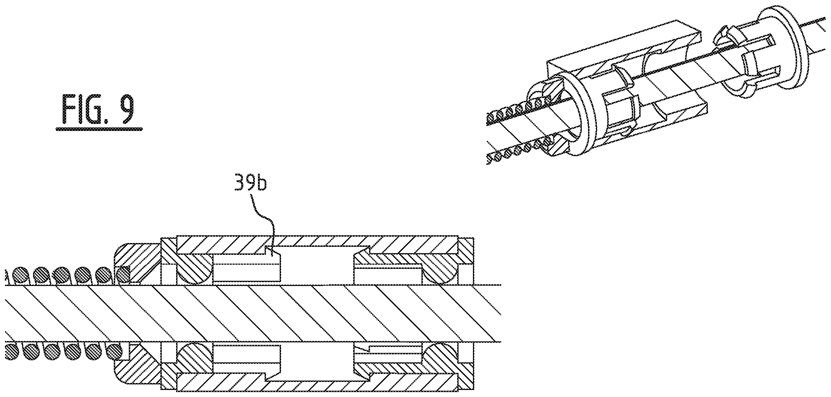

Figure 9 illustrates a cross-section of the connector with a connector body 10 having a snap fit mechanism for locking bearing holders 35. Each bearing holder 35 is provided with a locking profile 39b for a snap fit with a complementary locking profile provided on the inner side of the body 10 such that the bearing holders 35 and the bearings 34 may be presented via the ends and snap fitted inside the body 10 when the locking profiles 39b on the bearing holder 35 and the body 10 interlock.

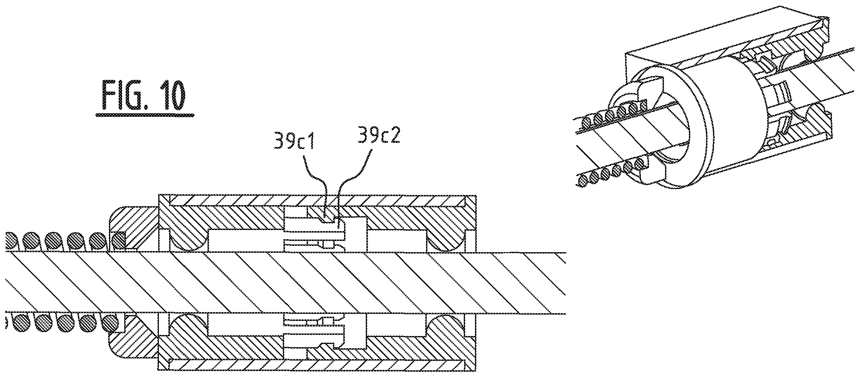

Figure 10 illustrates a cross-section of the connector with a connector body 10 configured for interlocking bearing holders 35. Bearing holders 35 on opposite ends of the body are configured to interlock inside the body via interlocking profiles 39c1, 39c2 such that the bearing holders 35 and the bearings 34 may be presented via the ends and snap fitted together inside the body 10 when the locking profiles39c of the bearing holders 35 interlock.

It is further noted that the above list of ways of mounting the bearings 34 inside the connector 10 is not exhaustive. Other alternatives may be envisaged by a skilled person depending on the circumstances and materials used. One can imagine for instance alternatives where the bearings would not be inserted by the ends of the tubular opening 11 but for instance via lateral openings and slots in the body 10.

As far as materials are concerned, the connector is preferably made of titanium and the rod of cobal-chromium (CoCr) to prevent titanium-on titanium friction and metal debris. Other materials suitable, such as for instance poly ethylene, for the above described movements may however be envisaged as well.

According to an example, a method for spinal correction is provided, comprising the steps of:
- Providing a rod 300 for attachment to the spine,
- Providing an anchoring device 200 for attachment to the spine
- Providing a connector 100 according to the invention for connection to the rod 300 and the anchoring device 200, and
- Further comprising the step of coupling the connector 100 to the rod 300.

A further step comprises coupling the connector (100) to the anchoring device (200).

The method preferably comprises a step of attaching the rod 300 to the spine 1000, for instance using anchoring elements 400 and 500, being bone screws or bone pins. Providing the anchoring device 200 may comprise attaching the anchoring device 200 to the spine 1000, in particular to at least one vertebra 100n and optionally to another vertebrae 100(n+1). The anchoring device 200 may be unitary with the connector 100, in the alternative the connector 100 can be coupled to the anchoring device 200.

The connector 100 is preferably coupled to the rod 300 by sliding the connector100, preferably using the guiding mechanism 30 as described above, over the rod 300. After connection to the anchoring device 200, the anchoring device 200 can be attached to the spine.

Alternatively the connector 100 can be attached to the anchoring device 200 once the anchoring device 200 is attached to the spine.

Surgery can be performed in particular through a posterior midline skin incision. The anchoring elements 400, 500, 220, 230 can be placed with the freehand technique. The rods 200 and 300 can be passed sub-fascially and contoured into the desired shape in both the coronal and sagittal plane.

It is noted that a surgeon may decide depending on circumstances to alter the sequence of the steps, and start with any one of the anchoring device, the rod and or the connector.

Whilst the principles of the invention have been set out above in connection with specific embodiments, it is understood that this description is merely made by way of example and not as a limitation of the scope of protection which is determined by the appended claims.

## Claims

1. Connector (100) for slidably connecting an anchoring device (200) to a rod (300) in a spinal correction system, comprising
- a body (10), wherein the body (10) has a first tubular opening (11),
- a coupling mechanism (20) for coupling the body (10) to the anchoring device (200),
- a guiding mechanism (30) provided in said first tubular opening (11) and arranged to guide the rod (300) in a sliding movement along a longitudinal direction (A) of the rod (300), **characterized in that** said guiding mechanism has at least two guiding portions (32, 33) provided at a mutual distance (d) in said first tubular opening (11) and is further arranged to prevent any movement, with respect to the rod (300), other than a translation along the longitudinal direction (A) of the rod (300) and a rotation around said longitudinal direction (A) of the rod (300).

2. Connector according to any of the above claims, wherein the at least two guiding portions (32, 33) are provided at the end parts of the first tubular opening (11).

3. Connector according to any of the above claims, wherein the first tubular opening (11) has a first internal diameter (D1), the guiding portions (32, 33) have a second internal diameter (D2), and the first internal diameter (D1) is larger than the second internal diameter (D2).

4. Connector according to any of the above claims, wherein each guiding portion (32, 33) has a bearing (34) and a bearing holder (35).

5. Connector according to any of the above claims, wherein the guiding portions (32, 33) are spherical bearings.

6. Connector according to any of the above claims, wherein the guiding portions (32, 33) are slid inside the body (10) through slot openings (12, 13) in the body (10).

7. Connector according to any of the above claims, wherein the guiding portions (32 , 33) are slid inside the body (10) via the ends of the first tubular opening (11).

8. Connector according to the previous claim, wherein the guiding portions (32, 33) are fixed using one of a pin mechanism (36), a thread mechanism (37), a bayonet mechanism (38), a snap fit mechanism (39).

9. Connector according to claim 4, wherein the bearing holders (32, 33) are fixed using one of a pin mechanism (35), a thread mechanism (36), a bayonet mechanism (37), a snap fit mechanism (38).

10. Connector according to any of the above claims, further comprising a spring (40) for constraining the translation of the rod (300) along its longitudinal direction (A).

11. Connector according to any of the above claims, wherein the coupling mechanism (20) has a second tubular opening (21) for receiving a stationary rod (210), said second tubular opening (21) having a direction (B) substantially parallel to the direction of the first tubular opening (11).

12. Connector according to the previous claim, wherein the coupling mechanism (20) has a locking mechanism (25) for locking the stationary rod (210) with respect to the coupling mechanism (20).

13. Connector assembly (100,200) for spinal correction comprising a connector (100) according to any of the above claims and an anchoring device (200) for anchoring to the spine, said anchoring device (200) being coupled to the connector (100) via the coupling mechanism (20) of the connector, wherein the anchoring device (200) preferably has a stationary rod (210) and two or more anchoring elements (220,230) for anchoring in one or more vertebrae, wherein the stationary rod (210) is fixed by two anchoring elements (220, 230) in two neighboring vertebrae.

14. Spinal correction system comprising a rod (300) for spinal correction and at least one connector (100) according to any of claims 1 - 12, preferably further comprising an anchoring device (200) to form with the at least one connector (100) a connector assembly (100, 200) according to claim 13, preferably further comprising one or more anchors (400, 500) for anchoring the rod (300) in the spine.

15. Kit of parts comprising at least two of a connector (100), an anchoring device (200), and a rod (300) according to any of the preceding claims.

## Patentansprüche

1. Verbinder (100) zum verschiebbaren Verbinden einer Verankerungsvorrichtung (200) mit einer Stange (300) in einem Wirbelsäulenkorrektursystem, umfassend
- einen Körper (10), wobei der Körper (10) eine erste röhrenförmige Öffnung (11) aufweist,
- einen Kopplungsmechanismus (20) zum Koppeln des Körpers (10) mit der Verankerungsvorrichtung (200),
- einen Führungsmechanismus (30), der in der ersten röhrenförmigen Öffnung (11) bereitgestellt und eingerichtet ist, um die Stange (300) in einer Schiebebewegung entlang einer Längsrichtung (A) der Stange (300) zu führen, **dadurch gekennzeichnet, dass** der Führungsmechanismus mindestens zwei Führungsabschnitte (32, 33) aufweist, die in einem gegenseitigen Abstand (d) in der ersten röhrenförmigen Öffnung (11) bereitgestellt sind, und ferner eingerichtet ist, um jegliche Bewegung in Bezug auf die Stange (300) außer einer Translation entlang der Längsrichtung (A) der Stange (300) und einer Drehung um die Längsrichtung (A) der Stange (300) herum zu verhindern.

2. Verbinder nach einem der vorstehenden Ansprüche, wobei die mindestens zwei Führungsabschnitte (32, 33) an den Endteilen der ersten röhrenförmigen Öffnung (11) bereitgestellt sind.

3. Verbinder nach einem der vorstehenden Ansprüche, wobei die erste röhrenförmige Öffnung (11) einen ersten Innendurchmesser (D1) aufweist, die Führungsabschnitte (32, 33) einen zweiten Innendurchmesser (D2) aufweisen und der erste Innendurchmesser (D1) größer als der zweite Innendurchmesser (D2) ist.

4. Verbinder nach einem der vorstehenden Ansprüche, wobei jeder Führungsabschnitt (32, 33) ein Lager (34) und einen Lagerhalter (35) aufweist.

5. Verbinder nach einem der vorstehenden Ansprüche, wobei die Führungsabschnitte (32, 33) sphärische Lager sind.

6. Verbinder nach einem der vorstehenden Ansprüche, wobei die Führungsabschnitte (32, 33) durch Schlitzöffnungen (12, 13) in dem Körper (10) in ein Inneres des Körpers (10) geschoben werden.

7. Verbinder nach einem der vorstehenden Ansprüche, wobei die Führungsabschnitte (32, 33) über die Enden der ersten röhrenförmigen Öffnung (11) in das Innere des Körpers (10) geschoben werden.

8. Verbinder nach dem vorstehenden Anspruch, wobei die Führungsabschnitte (32, 33) unter Verwendung eines von einem Stiftmechanismus (36), einem Gewindemechanismus (37), einem Bajonettmechanismus (38) oder einem Schnappmechanismus (39) befestigt sind.

9. Verbinder nach Anspruch 4, wobei die Lagerhalter (32, 33) unter Verwendung eines von einem Stiftmechanismus (35), einem Gewindemechanismus (36), einem Bajonettmechanismus (37) oder einem Schnappmechanismus (38) befestigt sind.

10. Verbinder nach einem der vorstehenden Ansprüche, ferner umfassend eine Feder (40) zum Beschränken der Translation der Stange (300) entlang ihrer Längsrichtung (A).

11. Verbinder nach einem der vorstehenden Ansprüche, wobei der Kopplungsmechanismus (20) eine zweite röhrenförmige Öffnung (21) zum Aufnehmen einer stationären Stange (210) aufweist, wobei die zweite röhrenförmige Öffnung (21) eine Richtung (B) aufweist, die im Wesentlichen parallel zu der Richtung der ersten röhrenförmigen Öffnung (11) ist.

12. Verbinder nach dem vorstehenden Anspruch, wobei der Kopplungsmechanismus (20) einen Verriegelungsmechanismus (25) zum Verriegeln der stationären Stange (210) in Bezug auf den Kopplungsmechanismus (20) aufweist.

13. Verbinderanordnung (100,200) für eine Wirbelsäulenkorrektur, umfassend einen Verbinder (100) nach einem der vorstehenden Ansprüche und eine Verankerungsvorrichtung (200) zum Verankern mit der Wirbelsäule, wobei die Verankerungsvorrichtung (200) über den Kopplungsmechanismus (20) des Verbinders mit dem Verbinder (100) gekoppelt ist, wobei die Verankerungsvorrichtung (200) vorzugsweise eine stationäre Stange (210) und zwei oder mehr Verankerungselemente (220,230) zum Verankern in einem oder mehreren Wirbeln aufweist, wobei die stationäre Stange (210) mittels zweier Verankerungselemente (220, 230) in zwei benachbarten Wirbeln befestigt ist.

14. Wirbelsäulenkorrektursystem, umfassend eine Stange (300) für die Wirbelsäulenkorrektur und mindestens einen Verbinder (100) nach einem der Ansprüche 1 bis 12, vorzugsweise ferner umfassend eine Verankerungsvorrichtung (200), um mit dem mindestens einen Verbinder (100) eine Verbinderanordnung (100, 200) nach Anspruch 13 auszubilden, vorzugsweise ferner umfassend einen oder mehrere Anker (400, 500) zum Verankern der Stange (300) in der Wirbelsäule.

15. Teilesatz, umfassend mindestens zwei von einem Verbinder (100), einer Verankerungsvorrichtung (200) und einer Stange (300) nach einem der vorstehenden Ansprüche.

## Revendications

1. Élément de liaison (100) permettant de relier de manière coulissante un dispositif d'ancrage (200) à une tige (300) dans un système de correction de colonne vertébrale, comprenant
- un corps (10), dans lequel le corps (10) a une première ouverture tubulaire (11),
- un mécanisme d'accouplement (20) pour accoupler le corps (10) au dispositif d'ancrage (200),
- un mécanisme de guidage (30) prévu dans ladite première ouverture tubulaire (11) et conçu pour guider la tige (300) dans un mouvement de coulissement le long d'une direction longitudinale (A) de la tige (300),
**caractérisé en ce que** ledit mécanisme de guidage a au moins deux parties de guidage (32, 33) prévues à une distance mutuelle (d) dans ladite première ouverture tubulaire (11) et est en outre agencé pour empêcher tout mouvement, par rapport à la tige (300), autre qu'une translation le long de la direction longitudinale (A) de la tige (300) et une rotation autour de ladite direction longitudinale (A) de la tige (300).

2. Élément de liaison selon l'une quelconque des revendications précédentes, dans lequel les au moins deux parties de guidage (32, 33) sont prévues au niveau des parties d'extrémité de la première ouverture tubulaire (11).

3. Élément de liaison selon l'une quelconque des revendications précédentes, dans lequel la première ouverture tubulaire (11) a un premier diamètre interne (D1), les parties de guidage (32, 33) ont un second diamètre interne (D2), et le premier diamètre interne (D1) est plus grand que le second diamètre interne (D2).

4. Élément de liaison selon l'une quelconque des revendications précédentes, dans lequel chaque partie de guidage (32, 33) a un palier (34) et un support de palier (35).

5. Élément de liaison selon l'une quelconque des revendications précédentes, dans lequel les parties de guidage (32, 33) sont des paliers à rotule.

6. Élément de liaison selon l'une quelconque des revendications précédentes, dans lequel les parties de guidage (32, 33) sont coulissées à l'intérieur du corps (10) à travers des ouvertures de fente (12, 13) dans le corps (10).

7. Élément de liaison selon l'une quelconque des revendications précédentes, dans lequel les parties de guidage (32, 33) sont coulissées à l'intérieur du corps (10) par l'intermédiaire des extrémités de la première ouverture tubulaire (11).

8. Élément de liaison selon la revendication précédente, dans lequel les parties de guidage (32, 33) sont fixées à l'aide de l'un parmi un mécanisme à goupille (36), un mécanisme à filetage (37), un mécanisme à baïonnette (38), un mécanisme à encliquetage (39).

9. Élément de liaison selon la revendication 4, dans lequel les supports de palier (32, 33) sont fixés à l'aide de l'un parmi un mécanisme à goupille (35), un mécanisme à filetage (36), un mécanisme à baïonnette (37), un mécanisme à encliquetage (38).

10. Élément de liaison selon l'une quelconque des revendications précédentes, comprenant en outre un ressort (40) pour contraindre la translation de la tige (300) le long de sa direction longitudinale (A).

11. Élément de liaison selon l'une quelconque des revendications précédentes, dans lequel le mécanisme d'accouplement (20) a une seconde ouverture tubulaire (21) pour recevoir une tige fixe (210), ladite seconde ouverture tubulaire (21) ayant une direction (B) sensiblement parallèle à la direction de la première ouverture tubulaire (11).

12. Élément de liaison selon la revendication précédente, dans lequel le mécanisme d'accouplement (20) a un mécanisme de verrouillage (25) pour verrouiller la tige fixe (210) par rapport au mécanisme d'accouplement (20).

13. Assemblage d'éléments de liaison (100, 200) permettant de corriger la colonne vertébrale comprenant un élément de liaison (100) selon l'une quelconque des revendications précédentes et un dispositif d'ancrage (200) permettant l'ancrage à la colonne vertébrale, ledit dispositif d'ancrage (200) étant accouplé à l'élément de liaison (100) par l'intermédiaire du mécanisme d'accouplement (20) de l'élément de liaison, dans lequel le dispositif d'ancrage (200) a de préférence une tige fixe (210) et deux éléments d'ancrage ou plus (220, 230) pour l'ancrage dans une ou plusieurs vertèbres, dans lequel la tige fixe (210) est fixée par deux éléments d'ancrage (220, 230) dans deux vertèbres voisines.

14. Système de correction de colonne vertébrale comprenant une tige (300) permettant de corriger la colonne vertébrale et au moins un élément de liaison (100) selon l'une quelconque des revendications 1 à 12, comprenant de préférence en outre un dispositif d'ancrage (200) pour former avec l'au moins un élément de liaison (100) un assemblage d'éléments de liaison (100, 200) selon la revendication 13, comprenant de préférence en outre un ou plusieurs ancrages (400, 500) pour ancrer la tige (300) dans la colonne vertébrale.

15. Kit de pièces comprenant au moins deux pièces parmi un élément de liaison (100), un dispositif d'ancrage (200) et une tige (300) selon l'une quelconque des revendications précédentes.
